# EUROPEAN PATENT SPECIFICATION

(11) **EP 2 694 065 B1**
(45) Date of publication and mention of the grant of the patent: **13.12.2017**
(21) Application number: 12768489.2
(22) Date of filing: 03.04.2012
(51) Int. Cl.: A61K 31/495, A61K 31/137, A61K 31/135, A61P 15/00, A61K 31/496, A61P 15/10, A61K 45/06, A61K 31/497

(54) **COMPOSITION FOR TREATING HYPOACTIVE SEXUAL DESIRE DISORDER**
STOFFGEMISCHE ZUR BEHANDLUNG DER STÖRUNG MIT VERMINDERTER SEXUELLER APPETENZ
COMPOSITION POUR LE TRAITEMENT DE LA BAISSE DU DÉSIR SEXUEL

(30) Priority: 04.04.2011 US 201161471505 P
(43) Date of publication of application: 12.02.2014
(73) Proprietor: S1 Biopharma, Inc., Jersey City, NJ 07305 (US)
(72) Inventor: SITCHON, Nicolas, G., Jersey City, NJ 07305 (US)
(74) Representative: Grund, Martin
(86) International application number: PCT/US2012/031991
(87) International publication number: WO 2012/138653

(56) References cited:
- WO-A1-2005/102342
- US-A1- 2006 211 685
- SCHENCK CARLOS H ET AL: "Combined bupropion-levodopa-trazodone therapy of sleep-related eating and sleep disruption in two adults with chemical dependency", SLEEP, ALLEN PRESS, LAWRENCE, KS, US, vol. 23, no. 5, 1 January 2000 (2000-01-01), pages 587(1)-588(2), XP008170655, ISSN: 0161-8105
- NIERENBERG A A ET AL: "TRAZODONE FOR ANTIDEPRESSANT-ASSOCIATED INSOMNIA", AMERICAN JOURNAL OF PSYCHIATRY, AMERICAN PSYCHIATRIC PUBLISHING, INC, US, vol. 151, no. 7, 1 July 1994 (1994-07-01), pages 1069-1072, XP009000336, ISSN: 0002-953X
- MODELL J G ET AL: "EFFECT OF BUPROPION-SR ON ORGASMIC DYSFUNCTION IN NONDEPRESSED SUBJECTS: A PILOT STUDY", JOURNAL OF SEX AND MARITAL THERAPY, BRUNNER-MAZEL, INC, US, vol. 26, no. 3, 1 July 2000 (2000-07-01), pages 231-240, XP008053246, ISSN: 0092-623X, DOI: 10.1080/00926230050084623
- SEGRAVES ROBERT TAYLOR ET AL: "Bupropion sustained release for the treatment of hypoactive sexual desire disorder in premenopausal women", JOURNAL OF CLINICAL PSYCHOPHARMACOLOGY,, vol. 24, no. 3, 1 June 2004 (2004-06-01), pages 339-342, XP009147325, ISSN: 0271-0749
- RAYMOND LANCE ET AL: "PRELIMINARY COMMUNICATION ORAL TRAZODONE AS EMPIRICAL THERAPY FOR ERECTILE DYSFUNCTION: A RETROSPECTIVE REVIEW", UROLOGY, vol. 46, no. 1, 1 January 1995 (1995-01-01), pages 117-120, XP055129617,
- TORDJMAN G: "TREATMENT OF INHIBITION SEXUAL DESIRE IN WOMAN BY TRAZODONE", CONTRACEPTION FERTILITE SEXUALITE, vol. 14, no. 10, 1986, pages 935-939, XP008170678, ISSN: 1165-1083
- SCANDROGLIO ET AL: "Ex vivo binding of flibanserin to serotonin 5-HT1A and 5-HT2A receptors.", PHARMACOLOGICAL RESEARCH, vol. 43, no. 2, 1 February 2001 (2001-02-01), pages 179-183, XP055130387, ISSN: 1043-6618, DOI: 10.1006/phrs.2000.0762
- MARILYN CRAVEN ET AL.: 'What are the alternatives to Nefazodone ?' CANADIAN PSYCHIATRIC ASSOCIATION BULLETIN vol. 36, no. 4, 2004, page 21, XP008171060
- ALEXANDRA ZOURKOVA ET AL.: 'The efficacy of bupropion and trazodone in the treatment of hypoactive sexual desire disorder and orgasm dysfunction in non-depressed women' CESKA A SLOVENSKA PSYCHIATRIE, PRAHA, CLS JEP. vol. 104, no. SUPP.2, 2008, page 1068, XP008170658
- RAMEZ GHANBARI ET AL.: 'Sustained administration of Trazodone enhances serotonergic neurotransmission: in vivo electrophysiological study in the rat brain' THE JOURNAL OF PHARMACOLOGY AND EXPERIMENTAL THERAPEUTICS vol. 335, no. 1, 2010, pages 197 - 206, XP055125766

## Description

### RELATED APPLICATION

This application claims the benefit of and priority to U.S. Provisional Patent Application No. 61/471,505, filed April 4, 2011.

### BACKGROUND OF THE INVENTION

Hypoactive sexual desire disorder (HSDD) is defined as the persistent or recurrent lack or absence of sexual fantasies and desire for sexual activity which causes marked distress or interpersonal difficulties and is not better accounted for by another mental disorder, a drug (legal or illegal), or some other medical condition. Synonyms for HSDD include sexual aversion, i.e., extreme aversion to, absence of, and avoidance of all, or almost all, sexual contact with a partner; inhibited sexual desire; sexual apathy; loss of libido; decreased sexual desire; distressing loss of sexual desire; and sexual anorexia. HSDD occurs in both sexes. It is considered to be the most common of all female sexual disorders, possibly occurring in as many as 10% of women in the United States.

Female Sexual Dysfunction (FSD) is described as an interruption in sexual functioning. The most common type of FSD is generalized, acquired HSDD defined by the DSM-IV-TR as: "The persistent lack (or absence) of sexual fantasies or desire for any form of sexual activity marked by distress or interpersonal difficulty and not better accounted for by another disorder (except another sexual dysfunction) direct physiological effects of a substance (including medications) or a general medical condition." The presence of distress or interpersonal difficulty is an integral part of HSDD and is central to the diagnosis of the condition. Approximately 1 in 10 women reported low sexual desire with associated distress, which may be HSDD.

A majority of HSDD cases are generalized in subtype, though a substantial minority of cases may relate to dissatisfaction or loss of interest in the sexual partner. Either subtype of HSDD can lead to general feelings of dissatisfaction in the person and/or discord in their personal relationships, including for example marital discord. HSDD, whether generalized or situational, often does not respond to counseling therapy, and frequently culminates in separation, finding a new sexual partner, and divorce.

Modell et al. (J. Sex & Marital Therapy 2000, 26:231-240) discusses a study in which sustained-release bupropion was used in nondepressed women and men having nonphysiologic orgasmic delay or inhibition. While Modell concludes that bupropion sustained release may be a useful agent in treating orgasmic inhibition and disorders of sexual arousal in men and women, Modell et al. nevertheless believes that a double-blind, placebo-controlled, random-assignment study protocol is necessary to provide a more complete separation of placebo from drug effects on sexual function as well as to elucidate further the effect of bupropion on specific portions of the sexual response cycle. Segraves et al. (J Clinical Psychopharmacology 2004, 24(3);339-342) reports that greater sexual responsiveness is observed in premonopausal women receiving bupropion sustained release based upon an outcome that was measured by investigator-rating and self-administered questionnaires.

Lance et al. (Urology 1995, 46(1): 117-120) is a retrospective review of patient-reported responses to trazodone was performed to outline the efficacy and side-effect profile of trazodone, While Lance et al. reports that trazodone appears to benefit younger male patients with erectile dysfunction with few known risk factors, Lance et al believes that a prospective, placebo-controlled trial is needed to confirm the observations of the pilot study on which the retrospective review was based. Tordjman reports (Contraception-fertilité-sexualité 1986, 14(10);935-939) reports that 30 female patients, with inhibited sexual desire (I.S.D.), were treated with trazodone. While Tordjman reports that 18 of the 30 women experienced a noticeable increase in their libido, coinciding for 6 of them with a remission of their depressive episode, 12 women did not report any improvement and, among those 12 women, 2 wome reported a remission of their depressive episode without noting any perceptible change in their libido.

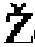 ourková *et al.* ( 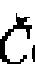 *eská a slovenská psychiatrie* 2008, 104S2:1068) report that trazodone and bupropion were each used separately to treat women with HSDD, and that their respective efficacies were about equal.

As there is no currently approved treatment for HSDD in the United States, a therapeutic composition and methods for ameliorating HSDD is an unmet need for a significant portion of the population and their quality of life. Delineated herein are compositions and methods of treatment that may be useful to address this unmet need based on heretofore unexpected properties possessed by the subject compositions.

### SUMMARY OF THE INVENTION

The invention is defined by the appended claims.

In one aspect, the invention provides compositions for use in treating a subject suffering from or susceptible to a sexual disorder or symptom thereof (e.g., female sexual dysfunction (FSD), hypoactive sexual desire disorder (HSDD), female orgasm disorder (FOD), female sexual arousal disorder (FSAD), sexual pain dysfunction, sexual interest arousal disorder, male sexual dysfunction (MSD), erectile disorder (ED)) wherein a therapeutically effective amount of a composition delineated herein is to be administered to a subject in need thereof. The composition comprises bupropion, trazodone, and a pharmaceutically acceptable carrier. In one aspect the composition is that comprising trazodone in a dosage range of 25-450 mg and bupropion in a dosage range of 200-450 mg. In another aspect the composition is that comprising trazodone in a dosage range of 25-450 mg and bupropion in a dosage range of 25-450 mg.

In one embodiment, the composition is that comprising bupropion, comprising bupropion in a dosage range of 200-450 mg; comprising bupropion in a dosage range of 225-300 mg; or comprising bupropion in a dosage range of 200-275 mg; comprising bupropion in a dosage range of 100-450 mg; comprising bupropion in a dosage range of 100-275 mg; comprising bupropion in a dosage range of 25-275 mg; comprising bupropion in a dosage range of XX-YY mg, wherein XX is an integer between 5 and 400 and YY is an integer between 50 and 450.

In one embodiment, the composition is that comprising trazodone, comprising trazodone in a dosage range of 25-450 mg; comprising trazodone in a dosage range of 75-150 mg; or comprising trazodone in a dosage range of 30-100 mg; comprising trazodone in a dosage range of XX- YY mg, wherein XX is an integer between 25 and 400 and YY is an integer between 50 and 450.

In one aspect, the invention provides a composition comprising bupropion, trazodone, and a pharmaceutically acceptable carrier for use in a method of treating a subject suffering from or susceptible to a hypoactive sexual desire disorder (HSDD), wherein a therapeutically effective amount of the composition is to be administered to a subject in need thereof.

In aspects, the method is that wherein the female sexual disorder (FSD) is HSDD. In aspects, the method is that wherein the FSD is female orgasm disorder (FOD); wherein the FSD is female sexual arousal disorder (FSAD); or wherein the FSD is sexual pain disorder or dysfunction, In aspects, the method is that wherein the FSD includes one or more simultaneous dysfunctions of sexual desire, arousal, orgasm, and/or pain. In aspects, the method is that wherein the male sexual disorder (MSD) is male HSDD or ED.

Thus, an aspect is a composition herein for use in treating erectile dysfunction (ED) in a subject.

Another aspect is a composition herein for use in treating male HSDD in a subject.

Other aspects include those, wherein the composition is administered orally; wherein the composition is administered topically; wherein the subject is diagnosed and being treated for depression; wherein the subject is not undergoing treatment for depression; wherein the subject is concurrently prescribed an additional therapeutic agent; or wherein the subject is concurrently not prescribed an additional therapeutic agent; wherein the subject is concurrently administered an additional therapeutic agent; or wherein the subject is concurrently not administered an additional therapeutic agent.

In one embodiment, the composition is that comprising bupropion, comprising bupropion in a dosage range of 100-450 mg qd; comprising bupropion in a dosage range of 200-450 mg qd; comprising bupropion in a dosage range of 100-300 mg qd; comprising bupropion in a dosage range of 225-300 mg qd; comprising bupropion in a dosage range of 100-275 mg qd; or comprising bupropion in a dosage range of 200-275 mg qd; comprising bupropion in a dosage range of XX-YY mg qd, wherein XX is an integer between 5 and 400 and YY is an integer between 50 and 450.

In one embodiment, the composition is that comprising trazodone, comprising trazodone in a dosage range of 25-450 mg qd; comprising trazodone in a dosage range of 75-150 mg qd; or comprising trazodone, in a dosage range of 50-100 mg qd; comprising trazodone in a dosage range of XX-YY mg qd, wherein XX is an integer between 25 and 400 and YY is an integer between 50 and 450.

In one embodiment, the composition is that comprising bupropion and trazodone, comprising bupropion in a dosage range of 50-450 mg and trazodone in a dosage range of 25-450 mg; comprising bupropion in a dosage range of 200-450 mg and trazodone in a dosage range of 25-450 mg; comprising bupropion in a dosage range of 100-300 mg qd and comprising trazodone in a dosage range of 75- 150 mg qd; comprising bupropion in a dosage range of 225-300 mg qd and comprising trazodone in a dosage range of 75-150 mg qd; comprising bupropion in a dosage range of 100-275 mg qd and comprising trazodone in a dosage range of 50-100 mg qd; or comprising bupropion in a dosage range of 200-275 mg qd and comprising trazodone in a dosage range of 50- 100 mg qd.

In one aspect, the invention provides a kit comprising a composition delineated herein for use in treating or ameliorating HSDD or symptoms thereof in a subject.

In another aspect, the invention provides bupropion and trazodone, for use in a method of treating hypoactive sexual desire disorder (HSDD) in a subject. The methods herein can further comprise those wherein the subject is identified as in need of such treatment, and those wherein the subject is treated upon administration of the compounds and/or compositions herein. The methods can include those wherein the subject has not previously been administered the compounds and/or compositions herein, or wherein the subject has not previously been administered the compounds and/or compositions herein at the stated dosage levels or administration regimens.

In aspects, the subject is that wherein the subject is not being treated with a selective serotonin reuptake inhibitor (SSRI) agent.

In aspects, the subject is that wherein the subject is being treated with a selective serotonin reuptake inhibitor (SSRI) agent.

In aspects, the subject is that wherein the subject is identified as having selective serotonin reuptake inhibitor (SSRI) agent induced HSDD.

In aspects, the subject is that wherein the subject is being treated with a PDE-5 inhibitor compound (i.e., sildenafil, tadalafil, and the like).

In aspects, the subject is that wherein the subject is not concurrently being treated with a PDE-5 inhibitor compound (i.e., sildenafil, tadalafil, and the like).

In aspects, the subject is that wherein the subject is being treated with an endocrine agent (e.g., testosterone).

In aspects, the subject is that wherein the subject is not concurrently being treated with an endocrine agent (e.g., testosterone).

In another aspect, the methods herein comprise taking a sample (i.e., fluid, blood, urine, saliva, tissue, etc.) and assessing a biological marker (i.e., liver enzymes, CYP3A4, and/or a genetic marker of the transport, receptor type, receptor density, receptor affinity, metabolism, or activity of serotonin, serotonin 1A or 2A subtype, dopamine, or a receptor subtype of dopamine) to measure health status of the subject either prior to, during or after administration of the compositions herein.

### DETAILED DESCRIPTION OF THE INVENTION

The invention is defined by the appended claims.

The present inventors have now discovered a therapeutic strategy that addresses hypoactive sexual desire disorder (HSDD) in a subject.

The present invention relates, at least in part, to the discovery that a combination of a 5-HT_{2A} antagonist (which is optionally a 5-HT_{1A} receptor agonist), a norepinephrine-dopamine reuptake inhibitor, (and optionally an endocrine active agent) provides unexpected superior and synergistic results in addressing hypoactive sexual desire disorder (HSDD) in a subject.

### 1. DEFINITIONS

Before further description of the present invention, and in order that the invention may be more readily understood, certain terms are first defined and collected here for convenience.

The term "administration" or "administering" includes routes of introducing the compound of the invention(s) to a subject to perform their intended function. Examples of routes of administration that may be used include injection (subcutaneous, intravenous, parenterally, intraperitoneally, intrathecal), oral, buccal, sublingual, inhalation, rectal and transdermal. The pharmaceutical preparations may be given by forms suitable for each administration route. For example, these preparations are administered in tablets or capsule form, by injection, inhalation, eye lotion, ointment, suppository, etc. administration by injection, infusion or inhalation; topical by lotion or ointment; and rectal by suppositories. Oral administration is preferred. The injection can be bolus or can be continuous infusion. Depending on the route of administration, the compound of the invention can be coated with or disposed in a selected material to protect it from natural conditions which may detrimentally effect its ability to perform its intended function. The compound of the invention can be administered alone, or in conjunction with either another agent as described above or with a pharmaceutically-acceptable carrier, or both. The compound of the invention can be administered prior to the administration of the other agent, simultaneously with the agent, or after the administration of the agent.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. The term alkyl further includes alkyl groups, which can further include oxygen, nitrogen, sulfur or phosphorous atoms replacing one or more carbons of the hydrocarbon backbone, e.g., oxygen, nitrogen, sulfur or phosphorous atoms. In preferred embodiments, a straight chain or branched chain alkyl has 30 or fewer carbon atoms in its backbone (e.g., C1-C30 for straight chain, C₃-C₃₀ for branched chain), preferably 26 or fewer, and more preferably 20 or fewer, and still more preferably 4 or fewer. Likewise, preferred cycloalkyls have from 3-10 carbon atoms in their ring structure, and more preferably have 3, 4, 5, 6 or 7 carbons in the ring structure.

Moreover, the term alkyl as used throughout the specification and sentences is intended to include both "unsubstituted alkyls" and "substituted alkyls," the latter of which refers to alkyl moieties having substituents replacing a hydrogen on one or more carbons of the hydrocarbon backbone. Such substituents can include, for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkylaryl, or an aromatic or heteroaromatic moiety. It will be understood by those skilled in the art that the moieties substituted on the hydrocarbon chain can themselves be substituted, if appropriate. Cycloalkyls can be further substituted, e.g., with the substituents described above. An "alkylaryl" moiety is an alkyl substituted with an aryl (e.g., phenylmethyl (benzyl)). The term "alkyl" also includes unsaturated aliphatic groups analogous in length and possible substitution to the alkyls described above, but that contain at least one double or triple bond respectively.

Unless the number of carbons is otherwise specified, "lower alkyl" as used herein means an alkyl group, as defined above, but having from one to ten carbons, more preferably from one to six, and still more preferably from one to four carbon atoms in its backbone structure, which may be straight or branched-chain. Examples of lower alkyl groups include methyl, ethyl, n-propyl, i-propyl, tert-butyl, hexyl, heptyl, octyl and so forth. In preferred embodiment, the term "lower alkyl" includes a straight chain alkyl having 4 or fewer carbon atoms in its backbone, e.g., C1-C4 alkyl.

The terms "alkoxyalkyl," "polyaminoalkyl" and "thioalkoxyalkyl" refer to alkyl groups, as described above, which further include oxygen, nitrogen or sulfur atoms replacing one or more carbons of the hydrocarbon backbone, e.g., oxygen, nitrogen or sulfur atoms.

The terms "alkenyl" and "alkynyl" refer to unsaturated aliphatic groups analogous in length and possible substitution to the alkyls described above, but that contain at least one double or triple bond, respectively. For example, the invention contemplates cyano and propargyl groups.

The term "aryl" as used herein, refers to the radical of aryl groups, including 5- and 6-membered single-ring aromatic groups that may include from zero to four heteroatoms, for example, benzene, pyrrole, furan, thiophene, imidazole, benzoxazole, benzothiazole, triazole, tetrazole, pyrazole, pyridine, pyrazine, pyridazine and pyrimidine, and the like. Aryl groups also include polycyclic fused aromatic groups such as naphthyl, quinolyl, indolyl, and the like. Those aryl groups having heteroatoms in the ring structure may also be referred to as "aryl heterocycles," "heteroaryls" or "heteroaromatics." The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings which are not aromatic so as to form a polycycle (e.g., tetralin).

The term "associating with" refers to a condition of proximity between a chemical entity or compound, or portions thereof, and a binding pocket or binding site on a protein. The association may be non-covalent (wherein the juxtaposition is energetically favored by hydrogen bonding or van der Waals or electrostatic interactions) or it may be covalent.

The language "biological activities" of a compound of the invention includes all activities elicited by compound of the inventions in a responsive cell. It includes genomic and non-genomic activities elicited by these compounds.

"Biological composition" or "biological sample" refers to a composition containing or derived from cells or biopolymers. Cell-containing compositions include, for example, mammalian blood, red cell concentrates, platelet concentrates, leukocyte concentrates, blood cell proteins, blood plasma, platelet-rich plasma, a plasma concentrate, a precipitate from any fractionation of the plasma, a supernatant from any fractionation of the plasma, blood plasma protein fractions, purified or partially purified blood proteins or other components, serum, semen, mammalian colostrum, milk, saliva, placental extracts, a cryoprecipitate, a cryosupernatant, a cell lysate, mammalian cell culture or culture medium, products of fermentation, ascites fluid, proteins induced in blood cells, and products produced in cell culture by normal or transformed cells (e.g., via recombinant DNA or monoclonal antibody technology). Biological compositions can be cell-free. In a preferred embodiment, a suitable biological composition or biological sample is a red blood cell suspension. In some embodiments, the blood cell suspension includes mammalian blood cells. Preferably, the blood cells are obtained from a human, a non-human primate, a dog, a cat, a horse, a cow, a goat, a sheep or a pig. In preferred embodiments, the blood cell suspension includes red blood cells and/or platelets and/or leukocytes and/or bone marrow cells.

The term "chiral" refers to molecules which have the property of non-superimposability of the mirror image partner, while the term "achiral" refers to molecules which are superimposable on their mirror image partner.

The term "diastereomers" refers to stereoisomers with two or more centers of dissymmetry and whose molecules are not mirror images of one another.

The term "effective amount" includes an amount effective, at dosages and for periods of time necessary, to achieve the desired result, e.g., sufficient to treat a hypoactive sexual desire disorder in a subject. An effective amount of compound of the invention may vary according to factors such as the disease state, age, and weight of the subject, and the ability of the compound of the invention to elicit a desired response in the subject. Dosage regimens may be adjusted to provide the optimum therapeutic response. An effective amount is also one in which any toxic or detrimental effects (e.g., side effects) of the compound of the invention are outweighed by the therapeutically beneficial effects.

A therapeutically effective amount of compound of the invention (i.e., an effective dosage) may range from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a compound of the invention can include a single treatment or, preferably, can include a series of treatments. In one example, a subject is treated with a compound of the invention in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of a compound of the invention used for treatment may increase or decrease over the course of a particular treatment. Administration regimens herein where designated are in accordance with the following abbreviations: SID or QD = Once a day; BID = Twice a day, TID = Three times a day; QID = Four times a day; q.h.s = every night.

The term "enantiomers" refers to two stereoisomers of a compound which are non-superimposable mirror images of one another. An equimolar mixture of two enantiomers is called a "racemic mixture" or a "racemate." The compounds of this invention may contain one or more asymmetric centers and thus occur as racemates and racemic mixtures, single enantiomers, individual diastereomers and diastereomeric mixtures. All such isomeric forms of these compounds are expressly included in the present invention. The compounds of this invention may also be represented in multiple tautomeric forms, in such instances, the invention expressly includes all tautomeric forms of the compounds described herein. All such isomeric forms of such compounds are expressly included in the present invention. All crystal forms of the compounds described herein are expressly included in the present invention.

The term "haloalkyl" is intended to include alkyl groups as defined above that are mono-, di- or polysubstituted by halogen, e.g., fluoromethyl and trifluoromethyl.

The term "halogen" designates -F, -Cl, -Br or -I.

The term "hydroxyl" means -OH.

The term "heteroatom" as used herein means an atom of any element other than carbon or hydrogen. Preferred heteroatoms are nitrogen, oxygen, sulfur and phosphorus.

The term "homeostasis" is art-recognized to mean maintenance of static, or constant, conditions in an internal environment.

The language "improved biological properties" refers to any activity inherent in a compound of the invention that enhances its effectiveness in vivo. In a preferred embodiment, this term refers to any qualitative or quantitative improved therapeutic property of a compound of the invention, such as reduced toxicity.

The term "optionally substituted" is intended to encompass groups that are unsubstituted or are substituted by other than hydrogen at one or more available positions, typically 1, 2, 3, 4 or 5 positions, by one or more suitable groups (which may be the same or different). Such optional substituents include, for example, hydroxy, halogen, cyano, nitro, C₁-C₈alkyl, C₂-C₈ alkenyl, C₂-C₈alkynyl, C₁-C₈alkoxy, C₂-C₈alkyl ether, C₃-C₈alkanone, C₁-C₈alkylthio, amino, mono- or di-(C1-C₈alkyl)amino, haloC₁-C₈alkyl, haloC₁-C₈alkoxy, C₁-C₈alkanoyl, C₂-C₈alkanoyloxy, C₁-C₈alkoxycarbonyl, -COOH, -CONH₂, mono- or di-(C₁ -C₈alkyl)aminocarbonyl, -SO₂NH₂, and/or mono or di(C₁-C₈alkyl)sulfonamido, as well as carbocyclic and heterocyclic groups. Optional substitution is also indicated by the phrase "substituted with from 0 to X substituents," where X is the maximum number of possible substituents. Certain optionally substituted groups are substituted with from 0 to 2, 3 or 4 independently selected substituents (i.e., are unsubstituted or substituted with up to the recited maximum number of substituents).

The term "isomers" or "stereoisomers" refers to compounds which have identical chemical constitution, but differ with regard to the arrangement of the atoms or groups in space.

The term "modulate" refers to an increase or decrease, e.g., the alteration in hypoactive sexual desire disorder and/or symptoms thereof in a subject such that a desired end result is achieved, e.g., a therapeutic result.

The term "obtaining" as in "obtaining a compound useful in treating hypoactive sexual desire disorder" is intended to include purchasing, synthesizing or otherwise acquiring the compound.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The terms "polycyclyl" or "polycyclic radical" refer to the radical of two or more cyclic rings (e.g., cycloalkyls, cycloalkenyls, cycloalkynyls, aryls and/or heterocyclyls) in which two or more carbons are common to two adjoining rings, *e.g.,* the rings are "fused rings". Rings that are joined through non-adjacent atoms are termed "bridged" rings. Each of the rings of the polycycle can be substituted with such substituents as described above, as for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkyl, alkylaryl, or an aromatic or heteroaromatic moiety.

The term "prodrug" or "pro-drug" includes compounds with moieties that can be metabolized *in vivo.* Generally, the prodrugs are metabolized *in vivo* by esterases or by other mechanisms to active drugs. Examples of prodrugs and their uses are well known in the art (See, *e.g.,* Berge et al. (1977) "Pharmaceutical Salts", J. Pharm. Sci. 66:1-19). The prodrugs can be prepared *in situ* during the final isolation and purification of the compounds, or by separately reacting the purified compound in its free acid form or hydroxyl with a suitable esterifying agent. Hydroxyl groups can be converted into esters *via* treatment with a carboxylic acid. Examples of prodrug moieties include substituted and unsubstituted, branch or unbranched lower alkyl ester moieties, (e.g., propionoic acid esters), lower alkenyl esters, di-lower alkyl-amino lower-alkyl esters (*e.g*., dimethylaminoethyl ester), acylamino lower alkyl esters (*e.g.,* acetyloxymethyl ester), acyloxy lower alkyl esters (e.g., pivaloyloxymethyl ester), aryl esters (phenyl ester), aryl-lower alkyl esters (*e.g.,* benzyl ester), substituted (*e.g.,* with methyl, halo, or methoxy substituents) aryl and aryl-lower alkyl esters, amides, lower-alkyl amides, di-lower alkyl amides, and hydroxy amides. Preferred prodrug moieties are propionoic acid esters and acyl esters. Prodrugs which are converted to active forms through other mechanisms *in vivo* are also included.

The language "a prophylactically effective amount" of a compound refers to an amount of a compound of the invention any formula herein or otherwise described herein which is effective, upon single or multiple dose administration to the patient, in preventing or treating a sexual disorder.

The language "reduced toxicity" is intended to include a reduction in any undesired side effect elicited by a compound of the invention when administered *in vivo.*

The term "subject" includes organisms which are capable of suffering from a sexual disorder or who could otherwise benefit from the administration of a compound or composition of the invention, such as human (male or female) and non-human animals (male or female). Preferred humans include human patients suffering from or prone to suffering from hypoactive sexual desire disorder or associated state, as described herein. The term "non-human animals" of the invention includes all vertebrates, *e.g.,* mammals, *e.g.,* rodents, *e.g.,* mice, and non-mammals, such as non-human primates, e.g., sheep, dog, cow, chickens, amphibians, reptiles, etc.

The term "susceptible to a hypoactive sexual desire disorder" is meant to include subjects at risk of developing hypoactive sexual desire disorder, e.g., subjects previously diagnosed as having or having a family or medical history of hypoactive sexual desire disorder.

The phrases "systemic administration,"administered systemically", "peripheral administration" and "administered peripherally" as used herein mean the administration of a compound of the invention(s), drug or other material, such that it enters the patient's system and, thus, is subject to metabolism and other like processes, for example, subcutaneous administration.

The language "therapeutically effective amount" of a compound of the invention of the invention refers to an amount of an agent which is effective, upon single or multiple dose administration to the patient, in modulating hypoactive sexual desire disorder and/or symptoms of hypoactive sexual desire disorder, or in improving the patient (either objectively or subjectively according to the patient or health care provider) beyond that expected in the absence of such treatment.

With respect to the nomenclature of a chiral center, terms "d" and "l" configuration are as defined by the IUPAC Recommendations. As to the use of the terms, diastereomer, racemate, epimer and enantiomer will be used in their normal context to describe the stereochemistry of preparations.

### 2. COMPOUNDS OF THE INVENTION

The compounds of the invention are defined in the claims. Further disclosed are other compounds capable of modulating hypoactive sexual desire disorder in a subject. Such compounds include a 5-HT_{2A} antagonist, a 5-HT_{1A} receptor agonist, a norepinephrine-dopamine reuptake inhibitor, and an endocrine active agent. Compositions of the invention further include a pharmaceutically acceptable carrier.

The compounds described herein include a 5-HT_{2A} antagonist, that is a compound that demonstrates antagonistic activity against the 5-HT_{2A} receptor; a norepinephrine-dopamine reuptake inhibitor; that is a compound that exhibits inhibition activity in norepinephrine-dopamine reuptake; a 5-HT_{1A} receptor agonist, that is a compound that demonstrates agonist activity against the 5-HT_{1A} receptor; and an endocrine active agent, that is an agent that is active in modulating the endocrine system. Also disclosed is a compound (e.g., a compound herein) capable of modulating hypoactive sexual desire disorder; and pharmaceutically acceptable esters, salts, isomers and prodrugs thereof.

Naturally occurring or synthetic isomers can be separated in several ways known in the art. Methods for separating a racemic mixture of two enantiomers include chromatography using a chiral stationary phase (see, *e.g.,* "Chiral Liquid Chromatography," W.J. Lough, Ed. Chapman and Hall, New York (1989)). Enantiomers can also be separated by classical resolution techniques. For example, formation of diastereomeric salts and fractional crystallization can be used to separate enantiomers. For the separation of enantiomers of carboxylic acids, the diastereomeric salts can be formed by addition of enantiomerically pure chiral bases such as brucine, quinine, ephedrine, strychnine, and the like. Alternatively, diastereomeric esters can be formed with enantiomerically pure chiral alcohols such as menthol, followed by separation of the diastereomeric esters and hydrolysis to yield the free, enantiomerically enriched carboxylic acid. For separation of the optical isomers of amino compounds, addition of chiral carboxylic or sulfonic acids, such as camphorsulfonic acid, tartaric acid, mandelic acid, or lactic acid can result in formation of the diastereomeric salts.

### 3. USES OF THE COMPOUNDS OF THE INVENTION

In one embodiment, the invention provides compounds for use in methods of treating hypoactive sexual desire disorder (HSDD) in a subject comprising administering to the subject a composition delineated herein. In certain embodiments, the subject is a mammal, e.g., a primate, e.g., a human. In aspect, the disease, disorder or symptom thereof in which the compounds, compositions, and methods of treatment relate to is one described in the Diagnostic and Statistical Manual of Mental Disorders 4th edition - Text Revision, (DSM-I-TRV), American Psychiatric Association.

In certain embodiments, a therapeutically effective amount of a compound of the invention may be administered in combination with another pharmaceutically active compound. Examples of pharmaceutically active compounds include compounds known to treat hypoactive sexual desire disorder (HSDD) in a subject. Other pharmaceutically active compounds that may be used can be found in Harrison's Principles of Internal Medicine, Thirteenth Edition, Eds. T.R. Harrison et al. McGraw-Hill N.Y., NY; and the Physicians Desk Reference 50th Edition 1997, Oradell New Jersey, Medical Economics Co. The compound of the invention and the pharmaceutically active compound may be administered to the subject in the same pharmaceutical composition or in different pharmaceutical compositions (at the same time or at different times).

Determination of a therapeutically effective hypoactive sexual desire disorder (HSDD) effective amount, a prophylactically effective hypoactive sexual desire disorder amount of the compound of the invention, can be readily made by the physician or veterinarian (the "attending clinician"), as one skilled in the art, by the use of known techniques and by observing results obtained under analogous circumstances. The dosages may be varied depending upon the requirements of the patient in the judgment of the attending clinician; the severity of the condition being treated and the particular compound being employed. In determining the therapeutically effective hypoactive sexual desire disorder amount or dose, and the prophylactically effective hypoactive sexual desire disorder amount or dose, a number of factors are considered by the attending clinician, including, but not limited to: the specific hypoactive sexual desire disorder involved; pharmacodynamic characteristics of the particular agent and its mode and route of administration; the desired time course of treatment; the species of mammal; its size, age, and general health; the specific disease involved; the degree of or involvement or the severity of the disease; the response of the individual patient; the particular compound administered; the mode of administration; the bioavailability characteristics of the preparation administered; the dose regimen selected; the kind of concurrent treatment (*i.e.,* the interaction of the compound of the invention with other co-administered therapeutics); and other relevant circumstances.

The dosage administration can be in a single dosage form or multiple dosage forms. The dosages can be administered concurrently, simultaneously, or sequentially. The dosages can be a single dosage immediately prior to sexual activity, or can be one or more doses daily without regard to timing prior to sexual activity. Treatment can be initiated with smaller dosages, which are less than the optimum dose of the compound. Thereafter, the dosage may be increased by small increments until the optimum effect under the circumstances is reached. For convenience, the total daily dosage may be divided and administered in portions during the day if desired. A therapeutically effective amount and a prophylactically effective amount of a compound of the invention of the invention is expected to vary from about 0.1 milligram per kilogram of body weight per day (mg/kg/day) to about 100 mg/kg/day.

The identification of those patients who are in need of prophylactic treatment for hypoactive sexual desire disorder is well within the ability and knowledge of one skilled in the art. Certain of the methods for identification of patients which are at risk of developing hypoactive sexual desire disorder which can be treated by the subject method are appreciated in the medical arts, such as family history, and the presence of risk factors associated with the development of that disease state in the subject patient (e.g., use of antidepressant drugs, hormonal contraceptives, antihormonal and/or cytotoxic chemotherapies, sedatives, antipsychotic drugs, antiepileptic drugs, mood stabilizer drugs, opioid drugs, alcohol, or narcotic drugs). A clinician skilled in the art can readily identify such candidate patients, by the use of, for example, clinical tests, physical examination and medical/family history.

As used herein, "obtaining a biological sample from a subject," includes obtaining a sample for use in the methods described herein. A biological sample is described above.

In another aspect, a compound of the invention is packaged in a therapeutically effective amount with a pharmaceutically acceptable carrier or diluent. The composition may be formulated for treating a subject suffering from or susceptible to a hypoactive sexual desire disorder, and packaged with instructions to treat a subject suffering from or susceptible to a hypoactive sexual desire disorder.

The subject may be at risk of a hypoactive sexual desire disorder, may be exhibiting symptoms of a hypoactive sexual desire disorder, may be susceptible to a hypoactive sexual desire disorder and/or may have been diagnosed with a sexual desire disorder.

If the modulation of the status indicates that the subject may have a favorable clinical response to the treatment, the subject may be treated with the compound. For example, the subject can be administered therapeutically effective dose or doses of the compound.

Kits of the invention include kits for treating a hypoactive sexual desire disorder in a subject. The kit may include the compounds of the invention and instructions for use. The instructions for use may include information on dosage, method of delivery, storage of the kit, etc. In aspects, the kits (and methods of using them) comprise instructions indicating that the compositions and/or treatment methods are contraindicated for (or not to be administered to) subjects that: (i) require and/or are taking CYP3A4, CYP 2B6-, or CYP 2D6-metabolized drugs; (ii) take any sex hormone other than an approved hormonal contraceptive; (iii) drink more than one alcoholic drink per day (e.g., 12-oz beer, 4-oz wine, etc).

Alternatively, the effects of compound of the invention can be characterized *in vivo* using animals models.

### 4. PHARMACEUTICAL COMPOSITIONS

The invention also provides a pharmaceutical composition, comprising an effective amount of a compound described herein and a pharmaceutically acceptable carrier. In a further embodiment, the effective amount is effective to treat a hypoactive sexual desire disorder, as described previously.

In an embodiment, the compound of the invention is administered to the subject using a pharmaceutically-acceptable formulation, *e.g.,* a pharmaceutically-acceptable formulation that provides sustained delivery of the compound of the invention to a subject for at least 12 hours, 24 hours, 36 hours, 48 hours, one week, two weeks, three weeks, or four weeks after the pharmaceutically-acceptable formulation is administered to the subject.

In certain embodiments, these pharmaceutical compositions are suitable for topical or oral, buccal or sublingual administration to a subject. In other embodiments, as described in detail below, the pharmaceutical compositions of the present invention may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound or composition herein.

The phrase "pharmaceutically acceptable" refers to those compound of the inventions of the present invention, compositions containing such compounds, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" includes pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier is "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Compositions containing a compound of the invention(s) include those suitable for oral, nasal, topical (including buccal and sublingual), rectal, vaginal, aerosol and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, more preferably from about 10 per cent to about 30 per cent.

Methods of preparing these compositions include the step of bringing into association a compound of the invention(s) with the carrier and, optionally, one or more accessory ingredients. In general, the formulations are prepared by uniformly and intimately bringing into association a compound of the invention with liquid carriers, or finely divided solid carriers, or both, and then, if necessary, shaping the product.

Compositions of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the invention(s) as an active ingredient. A compound may also be administered as a bolus, electuary or paste.

In solid dosage forms of the invention for oral administration (capsules, tablets, pills, dragees, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered active ingredient moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compound of the invention(s) include pharmaceutically-acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

In addition to inert diluents, the oral compositions can include adjuvants such as wetting agents, emulsifying and suspending agents, sweetening, flavoring, coloring, perfuming and preservative agents.

Suspensions, in addition to the active compound of the invention(s) may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions of the invention for rectal or vaginal administration may be presented as a suppository, which may be prepared by mixing one or more compound of the invention(s) with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Compositions of the present invention which are suitable for vaginal administration also include pessaries, tampons, creams, gels, pastes, foams or spray formulations containing such carriers as are known in the art to be appropriate.

Dosage forms for the topical or transdermal administration of a compound of the invention(s) include powders, sprays, ointments, pastes, creams, lotions, gels, solutions, patches and inhalants. The active compound of the invention(s) may be mixed under sterile conditions with a pharmaceutically-acceptable carrier, and with any preservatives, buffers, or propellants which may be required.

The ointments, pastes, creams and gels may contain, in addition to compound of the invention(s) of the present invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of the invention(s), excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The compound of the invention(s) can be alternatively administered by aerosol. This is accomplished by preparing an aqueous aerosol, liposomal preparation or solid particles containing the compound. A nonaqueous (*e.g.,* fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically-acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of a compound of the invention(s) to the body. Such dosage forms can be made by dissolving or dispersing the agent in the proper medium. Absorption enhancers can also be used to increase the flux of the active ingredient across the skin. The rate of such flux can be controlled by either providing a rate controlling membrane or dispersing the active ingredient in a polymer matrix or gel.

Ophthalmic formulations, eye ointments, powders, solutions and the like, are also contemplated as being within the scope of the invention.

Pharmaceutical compositions of the invention suitable for parenteral administration comprise one or more compound of the invention(s) in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers, which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of compound of the invention(s) in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

When the compound of the invention(s) are administered as pharmaceuticals, to humans and animals, they can be given per se or as a pharmaceutical composition containing, for example, 0.1 to 99.5% (more preferably, 0.5 to 90%) of active ingredient in combination with a pharmaceutically-acceptable carrier.

Regardless of the route of administration selected, the compound of the invention(s), which may be used in a suitable hydrated form, and/or the pharmaceutical compositions of the present invention, are formulated into pharmaceutically-acceptable dosage forms by conventional methods known to those of skill in the art.

Actual dosage levels and time course of administration of the active ingredients in the pharmaceutical compositions of the invention may be varied so as to obtain an amount of the active ingredient which is effective to achieve the desired therapeutic response for a particular patient, composition, and mode of administration, without being toxic to the patient. An exemplary dose range is from 0.1 to 10 mg per day.

A preferred dose of the compound of the invention for the present invention is the maximum that a patient can tolerate and not develop serious side effects. Preferably, the compound of the invention of the present invention is administered at a concentration of about 0.001 mg to about 100 mg per kilogram of body weight, about 0.001 - about 10 mg/kg or about 0.001 mg - about 100 mg/kg of body weight. Ranges intermediate to the above-recited values are also intended to be part of the invention.

### EXAMPLES

The invention is further illustrated by the following examples which are intended to illustrate but not limit the scope of the invention.

### MATERIALS

***Small-Molecule Compounds -*** bupropion, trazodone and testosterone (and their salt, solvates, hydrates, isomers, enantiomers, diasteriomers, racemates; all of which are included herein) are available from commercial sources and/or readily synthesized using methods and reagents know in the art. Bupropion is also known as, i.e., β-Keto-3-chloro-N-tert-butylamphetamine, i.e., (±)-2-(tert-Butylamino)-1-(3-chlorophenyl)propan-1-one; trazodone is also known as, i.e., 2-{3-[4-(3-chlorophenyl)piperazin-1-yl]propyl}[1,2,4]triazolo[4,3-a]pyridin-3(2H)-one.

### EXAMPLE 1

***Clinical protocol -*** subjects in a single blind, sequential study are administered bupropion and trazodone in increasing dosages @ 3 - 4 weeks each, that is, from a 3 (or 4) -week placebo baseline, to an intermediate dose (@ another 3-4 weeks), to a maximum dose (@ a final 3-4 weeks). The subjects' feedback/reports on subjective (e.g., feelings, sensations, general response) and objective (e.g., response time, performance measures, partner response) is collated and analyzed against dosage. Each study also includes one or more patient(s) serving as a control (in demonstrating the synergistic effect between the two actives) would receive only bupropion, while the second and third will each be given a different fixed dose combination products having a defined ratio of active ingredients (e.g., bupropion and trazodone).

### EXAMPLE 2

**Method.** A 36 year-old healthy male volunteer in a stable marital relationship for two years with no current sexual disorders, exposed himself sequentially to four treatments, each for 4 weeks: (1) Treatment B: Instant-release (IR) Bup 150 mg in the morning and 100 mg in the evening; (2) Treatment T: IR Trz 50 mg t.i.d.; (3) Treatment L_{low}, IR Trz 25 mg b.i.d. plus IR Bup 150 mg in the morning and 100 mg in the evening; and (4) Treatment L_{high}, IR Trz 50 mg t.i.d. plus IR Bup 75 mg t.i.d. A washout of 1-4 weeks occurred between each treatment. Level and frequency of sexual desire was scored daily, as not improved (0), somewhat improved (1), or markedly improved (2). Sexual events were counted, and three domains (sexual arousal, orgasm, and overall satisfaction with the event) were scored. Each of the sexual event variables was converted to a simple patient's global impression of improvement (PGI; improved or not improved today compared to pre-treatment baseline). The 3 domains of sexual event improvements were summed for analysis. Bup is already recommended as a treatment for HSDD; Trz is not. Thus, Fisher's exact test was applied post-hoc to the PGIs for Treatment B vs. Treatment L_{low} (L_{low}) and vs. Treatment L_{high} (L_{high}).

**Results.** For sexual desire, the mean score with L_{low} and L_{high} was about twice that with Treatment B (two-tailed paired t-test, p<0.0001), and Treatment B was superior to Treatment T. For arousal, orgasm, and overall satisfaction with a sexual event, L_{low} was associated with somewhat more improvements than with Treatment B in the third and fourth weeks of use. L_{high} was associated with significantly more improvements than with Treatment B in the third and fourth weeks of use and in the total for all four weeks. Fisher's exact test, two-tailed, showed the combination of bupropion plus trazodone superior, p<0.05, for the 3-domain sum of sexual event improvement. This study conducted with the combination of bupropion plus trazodone showed increased benefits in sexual arousal, orgasm, and event satisfaction, after exposure for 4 weeks, compared to either bupropion alone or trazodone alone. The effects occurred at or below the target dosage of bupropion or trazodone in their current (antidepressant) labeling.

An independent researcher then scored the desire results as unimproved = 0, somewhat improved = 1, and markedly improved = 2. Upon the advice of the independent researcher the subject dichotomized his sexual event results in a simple daily patient's global impression of improvement: improved or not improved today (compared to pre-treatment baseline). The independent researcher, when told that the results were positive for the subject but before seeing any of the data, decided to apply categorical tests to the most obvious comparisons between treatments: for the first two weeks, the second two weeks, and for all four weeks of treatment, low-dose combination of bupropion plus trazodone vs. corresponding dose of bupropion; and high-dose the combination of bupropion plus trazodone vs. same dose of trazodone. The test used for the desire score was a two-tailed paired t test. The test used for the two-category variables was an online two-tailed Fisher's exact test. Both were from the website graphpad.com. *
*http://graphpad.com/quickcalcs/chisquaredl.cfm

**Table. Scores of desire, and counts of sexual event domain improved per treatment**

| | 0-2 Daily Desire Score, total, % of max. (max.=28), ***mean ± SD,p-value vs. Bup¹*** | PGI Improved for Sexual Events, Sum of 3 domains³ | |
|---|---|---|---|
| Treatment | | n/N,% improved | p-value vs. Bup² |
| Bup wks 1-2 | 9, 32% ***0.65±0.74*** | 4/15, 27% | |
| Bup wks 3-4 | 14, 50% | 2/15, 13% | |
| Bup total | ***0***.***82±0***.***55*** | 6/30, 20% | |
| wks 1-2 | 2, 7% ***0.14±0.53 p=0.051*** | 3/12, 25% | n.s. |
| Trz wks 3-4 | 0, 0% | 3/18, 17% | |
| Trz total | 2, 4% ***0.07+0.38 p<0.0001*** | 6/30, 20% | n.s. |
| L_{low} wks 1-2 | 14, 50% ***0.64±0.74,** n.s.* | 3/15, 20% | n.s. |
| L_{low} wks 3-4 | 28, 100% | 11/21, 52% | |
| L_{low} total | ***1.50 ± 0.88 P<0.0001*** | 14/36, 39% | n.s. |
| L_{high} wks1-2 | 20, 71% ***1.43±0.94 p=0.021*** | 6/15, 40% | n.s. |
| L_{high} wks3-4 | 28, 100% | 13/18, 72% | |
| L_{high} total | ***1.71+0.71, p<0.0001*** | 20/33, 61% | 0.0019 |

| | | | |
|---|---|---|---|
| 1. P-values vs. corresponding treatment Bup, paired t test, two-tailed 2. P-values vs. corresponding treatment Bup, two-tailed Fisher's exact test 3. Sum of n improved in arousal, orgasm, and overall satisfaction Note: P-values larger than 0.1 are omitted from the table. B or Bup is bupropion alone, T or Trz is trazodone alone, L_{low} is the lower dose of Bup/Trz combination, L_{high} is the higher dose of Bup/Trz combination. | | | |

For sexual desire, the response to T (Treatment T) was low, to B (Treatment B) was intermediate, and to L_{low} and L_{high} was sometimes strong in the first two weeks and uniformly strong (improvement rated as marked every day) in the second two weeks of treatment. The differences between each dose of L vs. B were highly statistically significant, p<0.0001.

B was markedly superior to T, p<0.05 for all comparisons.

For the sum of improvements in arousal, orgasm, and overall satisfaction with a sexual event, L_{low} showed significantly more improvements than with B in the third and fourth weeks of use, 52% vs. 20%, p<0.05. L_{high} was associated with significantly more improvements than with B in the third and fourth weeks of use (72% vs. 13%) and in the total for all four weeks (61% vs. 20%). Fisher's exact test, two-tailed, showed the combination of bupropion plus trazodone superior, p<0.05, for each of these 3-domain sums of sexual event improvement.

For improvements in orgasm or overall satisfaction for a sexual event, the numbers appeared too small and the numerical trends were generally too weak to show statistically significant differences. For arousal, however, L_{high} was associated with significantly more improvements than with B in the third and fourth weeks of use (100% vs. 0%) and in the total for all four weeks (91% vs. 20%). Fisher's exact test, two-tailed, showed the combination of bupropion plus trazodone superior, p<0.05, for each of these 3-domain sums of sexual event improvement. A numerical trend also favored L_{high} in weeks 1-2 by 4/5 vs. 2/5 (80% vs. 40%).

The applicability of these male results to female subjects with HSDD is possible given the similarities of desire dysfunction in men and women [Laumann 1999], and is to be tested next.

### EXAMPLE 3

**Additional Study Design.** Further study is conducted as delineated in the Schematic below.

**Schematic of Study Design**

| Week | 0 | 1 | 2 | 3 | 4 | 5 |
|---|---|---|---|---|---|---|
| Day | 1-7 | 8-15 | 15-21 | 22-29 | 29-35 | 36 |
| Period | Screening | First Dosing | Washout | Second Dosing | Washout | Final Evaluations |
| Group One | X | Low dose drug B+ Low dose drug T | X | High dose drug B + High dose drug T | X | X |
| Group Two | X | High dose drug B | X | High dose drug T | X | X |

| | | | | | | |
|---|---|---|---|---|---|---|
| ***B or BUP*= *SR bupropion*** ***T or TRZ* = *SR trazodone*** | | | | | | |

**Flow Chart of Study Data Collection**

| Period | Screening | First and 8th day of each Treatment | Final Evaluation |
|---|---|---|---|
| Note: each subject undergoes 2 treatments, for 1 week each followed by a 1-week washout | Final Evaluation | | |
| Informed consent | X | | |
| FSFI-1wk recall; FSDS-R-1wk recall Both are self-rated (s) | X | X | X |
| Psychiatric history [clinician-rated (c)] | X | | |
| Relational/marital history (c) | X | | |
| PHQ-9 [self-rated (s)] | X | | |
| Beck Anxiety Inventory (s) | X | | |
| Sexual Interest and Desire Inventory - F (c) | X | | |
| Checklist for DSM-IV & DSM-5 female sexual disorders; FSD diagnoses (c) | X | | |
| Marital Adjustment Test (MAT) (s) | X | | X |
| Physical examination | X | | P.r.n. only |
| Laboratory analytes | X | | X |
| ECG, 12-lead | X | | X |
| Sexual Activity Log (s) | X | X | X |
| Sexual Desire Relationship Distress Scale (SDRDS) (s) if available from authors | X | X | X |
| Vital signs (supine and standing b.p., pulse)2 | X | Pre-dose & 1, 2, 4, 8 & 24 hr post-dose | X |
| AE inquiry and checklist2 | | | |
| Drug blood levels | | Pre-dose and 1, 2, 4, 6, 8, 12, 24 hr post-dose | |
| Cognitive test battery | | Pre-dose and 1, 2, and 4 hours post-dose | |
| Verbal Numeric Rating Scales of 6 feeling states (s) | X | | |
| Partner's tests (may do at home if use HTS) | **IIEF MAT (p) SDRDS (p)** | | **IIEF MAT (p) SDRDS (p)** |

Week 0: Informed consent, screening evaluations [Medical, psychiatric, social/relationship, and sexual history; diagnostics], measures of sexual dysfunction, and safety evaluations [physical examination, ECG, standard laboratory safety analytes]
Week 1: Treatment #1

### Group 1

Low Dose combination of bupropion plus trazodone: 250 mg BUP + 75 mg TRZ/day, given as 150 mg SR BUP in the morning and 100 mg SR BUP in the evening and 75 mg SR trazodone q.d.; and test battery. The test battery includes single-dose PK, steady-state PK and pharmacodynamics. Pharmacodynamics includes a cognitive test battery and numeric rating scales (NRS) of feeling states, which will be done in the morning of the first and last day of dosing, at pre-dose and at 1, 2, 4 and 8 hours post-dose. The cognitive testing battery includes choice reaction time, word recall, picture recognition, numeric and spatial working memory. The self-rated NRS of feeling states for sedation/activation includes drowsy, dizzy, nervous, agitated, and hyper. Cognitive testing will be done within -20 minutes before the hour; blood sampling will be done exactly on the hour; and VAS will be done within +15 minutes after the hour.
Or

### Group 2

150 mg SR BUP in the morning and 100 mg SR BUP in the evening
Week 2: washout #1
Week 3: Treatment #2:

### Group 1

High Dose combination of bupropion plus trazodone (250 mg BUP + 150 mg TRZ/day, given as 150 mg SR BUP in the morning and 100 mg SR BUP in the evening and 150 mg SR trazodone q.d.) and test battery
or

### Group 2

150 mg SR trazodone q.d.
Week 4: Washout #2

### EXAMPLE 4

Compositions of the invention can be made by combining the active agents (i.e., bupropion and trazodone) with one or more of the following exipients:
CARNAUBA WAX, CYSTEINE HYDROCHLORIDE, HYPROMELLOSES, MAGNESIUM STEARATE, CELLULOSE, MICROCRYSTALLINE, POLYETHYLENE, GLYCOL, POLYSORBATE 80, TITANIUM DIOXIDE, FD&C BLUE NO. 1;

Hydroxypropyl distarch phosphate (Contramid®), Hypromellose, Sodium stearyl fumarate, Colloidal silicon dioxide, Iron Oxide Yellow, Iron Oxide Red, Talc, Polyethylene Glycol 3350, Titanium Dioxide, Polyvinyl Alcohol, Black ink (food grade).

## Claims

1. A composition comprising trazodone, bupropion, and a pharmaceutically acceptable carrier for use in the treatment of female sexual dysfunction (FSD).

2. The composition for use according to claim 1, wherein the FSD is female orgasm disorder (FOD).

3. The composition for use according to claim 1, wherein the FSD is female sexual arousal disorder (FSAD).

4. The composition for use according to claim 1, wherein the FSD is sexual pain dysfunction.

5. The composition for use according to claim 1, wherein the FSD is hypoactive sexual desire disorder (HSDD)

6. A composition comprising trazodone, bupropion, and a pharmaceutically acceptable carrier for use in the treatment of male sexual dysfunction (MSD) in a subject.

7. A composition comprising trazodone, bupropion, and a pharmaceutically acceptable carrier for use in the treatment of erectile dysfunction (ED) in a subject.

8. A composition comprising trazodone, bupropion, and a pharmaceutically acceptable carrier for use in the treatment of sexual interest arousal disorder in a subject.

9. The composition for the use according to any one of claims 1-8, comprising trazodone in a dosage range of 25-450 mg and bupropion in a dosage range of 50-450 mg.

10. The composition for the use according to any one of claims 1-8, comprising trazodone in a dosage range of 25-450 mg and bupropion in a dosage range of 200-450 mg.

11. The composition for the use according to any one of claims 1-8, comprising trazodone in a dosage range of 75-150 mg qd and bupropion in a dosage range of 100-300 mg qd.

12. The composition for the use according to any one of claims 1-8, comprising trazodone in a dosage range of 50-100 mg qd and bupropion in a dosage range of 100-275 mg qd.

13. The composition for the use according to any one of claims 1-8, comprising trazodone in a dosage range of 75-150 mg qd and bupropion in a dosage range of 225-300 mg qd.

14. The composition for the use according to any one of claims 1-8, comprising trazodone in a dosage range of 50-100 mg qd and bupropion in a dosage range of 200-275 mg qd.

15. The composition for the use according to any one of claims 1-8, comprising trazodone in a dosage range of 25-450 mg.

16. The composition for the use according to any one of claims 1-15, wherein the composition is suitable for oral administration.

17. The composition for the use according to any one of claims 1-15, wherein the composition is suitable for topical administration.

18. The composition for use according to any one of claims 1-15, wherein the composition is administered orally.

19. The composition for use according to any one of claims 1-15, wherein the composition is administered topically.

20. The composition for use according to any one of claims 1-15, wherein the subject is diagnosed and being treated for depression.

21. The composition for use according to any one of claims 1-15, wherein the subject is not undergoing treatment for depression.

22. The composition for use according to any one of claims 1-15, wherein the subject is concurrently prescribed an additional therapeutic agent.

23. The composition for use according to any one of claims 1-15, wherein the subject is concurrently not prescribed an additional therapeutic agent.

24. An extended release composition comprising bupropion, trazodone, and a pharmaceutically acceptable carrier, for use in the treatment of female sexual dysfunction (FSD), hypoactive sexual desire disorder (HSDD), female orgasm disorder (FOD), sexual interest arousal disorder (FSAD), sexual pain dsyfunction, or ED in a subject.

25. A kit comprising a composition comprising
bupropion, trazodone, and a pharmaceutically acceptable carrier, and
a label providing instructions for administration of the composition to a subject,
for use in treating or ameliorating hypoactive sexual desire disorder (HSDD) or symptoms thereof in the subject.

26. A single dosage composition comprising bupropion, trazodone, and a pharmaceutically acceptable carrier, for use in the treatment of female sexual dysfunction (FSD), hypoactive sexual desire disorder (HSDD), female orgasm disorder (FOD), sexual interest arousal disorder (FSAD), sexual pain dysfunction, or ED in a subject.

27. Bupropion and trazodone for use in the treatment of female sexual dysfunction (FSD) in a subject.

28. Bupropion and trazodone for use in the treatment of hypoactive sexual desire disorder (HSDD) in a subject.

29. Bupropion and trazodone for use in the treatment of female orgasm disorder (FOD) in a subject.

30. Bupropion and trazodone for use in the treatment of sexual interest arousal disorder (FSAD) in a subject.

31. Bupropion and trazodone for use in the treatment of sexual pain dysfunction in a subject.

32. Bupropion and trazodone for use in the treatment of erectile dysfunction (ED) in a subject.

33. Bupropion and trazodone for use in the treatment of male sexual dysfunction (MSD) in a subject.

## Patentansprüche

1. Eine Zusammensetzung umfassend Trazodon, Bupropion und einen pharmazeutisch annehmbaren Träger zur Verwendung in der Behandlung von weiblicher sexueller Dysfunktion (FSD).

2. Die Zusammensetzung zur Verwendung gemäß Anspruch 1, wobei die FSD weibliche Orgasmusstörung (FOD) ist.

3. Die Zusammensetzung zur Verwendung gemäß Anspruch 1, wobei die FSD weibliche sexuelle Erregungsstörung (FSAD) ist.

4. Die Zusammensetzung zur Verwendung gemäß Anspruch 1, wobei die FSD sexuelle Schmerz-Dysfunktion ist.

5. Die Zusammensetzung zur Verwendung gemäß Anspruch 1, wobei die FSD vermindertes sexuelles Verlangen (HSDD) ist.

6. Eine Zusammensetzung umfassend Trazodon, Bupropion und einen pharmazeutisch annehmbaren Träger zur Verwendung in der Behandlung von männlicher sexueller Dysfunktion (MSD) in einem Subjekt.

7. Eine Zusammensetzung umfassend Trazodon, Bupropion und einen pharmazeutisch annehmbaren Träger zur Verwendung in der Behandlung von Erektionsstörung (ED) in einem Subjekt.

8. Eine Zusammensetzung umfassend Trazodon, Bupropion und einen pharmazeutisch annehmbaren Träger zur Verwendung in der Behandlung von Störung der Erregung des sexuellen Interesses in einem Subjekt.

9. Die Zusammensetzung zur Verwendung gemäß irgendeinem der Ansprüche 1-8, umfassend Trazodon in einem Dosierungsbereich von 25-450 mg und Bupropion in einem Dosierungsbereich von 50-450 mg.

10. Die Zusammensetzung zur Verwendung gemäß irgendeinem der Ansprüche 1-8, umfassend Trazodon in einem Dosierungsbereich von 25-450 mg und Bupropion in einem Dosierungsbereich von 200-450 mg.

11. Die Zusammensetzung zur Verwendung gemäß irgendeinem der Ansprüche 1-8, umfassend Trazodon in einem Dosierungsbereich von 75-150 mg qd und Bupropion in einem Dosierungsbereich von 100-300 mg qd.

12. Die Zusammensetzung zur Verwendung gemäß irgendeinem der Ansprüche 1-8, umfassend Trazodon in einem Dosierungsbereich von 50-100 mg qd und Bupropion in einem Dosierungsbereich von 100-275 mg qd.

13. Die Zusammensetzung zur Verwendung gemäß irgendeinem der Ansprüche 1-8, umfassend Trazodon in einem Dosierungsbereich von 75-150 mg qd und Bupropion in einem Dosierungsbereich von 225-300 mg qd.

14. Die Zusammensetzung zur Verwendung gemäß irgendeinem der Ansprüche 1-8, umfassend Trazodon in einem Dosierungsbereich von 50-100 mg qd und Bupropion in einem Dosierungsbereich von 200-275 mg qd.

15. Die Zusammensetzung zur Verwendung gemäß irgendeinem der Ansprüche 1-8, umfassend Trazodon in einem Dosierungsbereich von 25-450 mg.

16. Die Zusammensetzung zur Verwendung gemäß irgendeinem der Ansprüche 1-15, wobei die Zusammensetzung zur oralen Verabreichung geeignet ist.

17. Die Zusammensetzung zur Verwendung gemäß irgendeinem der Ansprüche 1-15, wobei die Zusammensetzung zur topischen Verabreichung geeignet ist.

18. Die Zusammensetzung zur Verwendung gemäß irgendeinem der Ansprüche 1-15, wobei die Zusammensetzung oral verabreicht wird.

19. Die Zusammensetzung zur Verwendung gemäß irgendeinem der Ansprüche 1-15, wobei die Zusammensetzung topisch verabreicht wird.

20. Die Zusammensetzung zur Verwendung gemäß irgendeinem der Ansprüche 1-15, wobei das Subjekt mit Depression diagnostiziert ist und behandelt wird.

21. Die Zusammensetzung zur Verwendung gemäß irgendeinem der Ansprüche 1-15, wobei das Subjekt nicht wegen Depression behandelt wird.

22. Die Zusammensetzung zur Verwendung gemäß irgendeinem der Ansprüche 1-15, wobei dem Subjekt zugleich ein zusätzliches therapeutisches Agens verschrieben ist.

23. Die Zusammensetzung zur Verwendung gemäß irgendeinem der Ansprüche 1-15, wobei dem Subjekt zugleich kein zusätzliches therapeutisches Agens verschrieben ist.

24. Eine Zusammensetzung zur verzögerten Freisetzung umfassend Bupropion, Trazodon und einen pharmazeutisch annehmbaren Träger zur Verwendung in der Behandlung von weiblicher sexueller Dysfunktion (FSD), vermindertem sexuellen Verlangen (HSDD), weiblicher Orgasmusstörung (FOD), Störung der Erregung des sexuellen Interesses (FSAD), sexueller Schmerz-Dysfunktion oder ED in einem Subjekt.

25. Ein Kit umfassend eine Zusammensetzung umfassend
Bupropion, Trazodon und einen pharmazeutisch annehmbaren Träger; und
ein Etikett mit Anweisungen zur Verabreichung der Zusammensetzung an ein Subjekt, zur Verwendung in der Behandlung oder Linderung von vermindertem sexuellen Verlangen (HSDD) oder Symptomen davon im Subjekt.

26. Eine Einzeldosis-Zusammensetzung umfassend Bupropion, Trazodon und einen pharmazeutisch annehmbaren Träger zur Verwendung in der Behandlung von weiblicher sexueller Dysfunktion (FSD), vermindertem sexuellen Verlangen (HSDD), weiblicher Orgasmusstörung (FOD), Störung der Erregung des sexuellen Interesses (FSAD), sexueller Schmerz-Dysfunktion oder ED in einem Subjekt.

27. Bupropion und Trazodon zur Verwendung in der Behandlung von weiblicher sexueller Dysfunktion (FSD) in einem Subjekt.

28. Bupropion und Trazodon zur Verwendung in der Behandlung von vermindertem sexuellem Verlangen (HSDD) in einem Subjekt.

29. Bupropion und Trazodon zur Verwendung in der Behandlung von weiblicher Orgasmusstörung (FOD) in einem Subjekt.

30. Bupropion und Trazodon zur Verwendung in der Behandlung von Störung der Erregung des sexuellen Interesses (FSAD) in einem Subjekt.

31. Bupropion und Trazodon zur Verwendung in der Behandlung von sexueller Schmerz-Dysfunktion in einem Subjekt.

32. Bupropion und Trazodon zur Verwendung in der Behandlung von Erektionsstörung (ED) in einem Subjekt.

33. Bupropion und Trazodon zur Verwendung in der Behandlung von männlicher sexueller Dysfunktion (MSD) in einem Subjekt.

## Revendications

1. Composition comprenant de la trazodone, du bupropion, et un support pharmaceutiquement acceptable à utiliser dans le traitement d'un dysfonctionnement sexuel féminin (FSD).

2. Composition à utiliser selon la revendication 1, dans laquelle le FSD est un trouble de l'orgasme féminin (FOD).

3. Composition à utiliser selon la revendication 1, dans laquelle le FSD est un trouble de l'excitation sexuelle féminine (FSAD).

4. Composition à utiliser selon la revendication 1, dans laquelle le FSD est une dyspareunie.

5. Composition à utiliser selon la revendication 1, dans laquelle le FSD est une baisse du désir sexuel (HSDD).

6. Composition comprenant de la trazodone, du bupropion, et un support pharmaceutiquement acceptable à utiliser dans le traitement d'un dysfonctionnement sexuel masculin (MSD) chez un sujet.

7. Composition comprenant de la trazodone, du bupropion, et un support pharmaceutiquement acceptable à utiliser dans le traitement d'un dysfonctionnement érectile (ED) chez un sujet.

8. Composition comprenant de la trazodone, du bupropion, et un support pharmaceutiquement acceptable à utiliser dans le traitement d'un trouble de l'excitation d'intérêt sexuel chez un sujet.

9. Composition à utiliser selon l'une quelconque des revendications 1 à 8, comprenant de la trazodone dans une plage posologique de 25 à 450 mg et du bupropion dans une plage posologique de 50 à 450 mg.

10. Composition à utiliser selon l'une quelconque des revendications 1 à 8, comprenant de la trazodone dans une plage posologique de 25 à 450 mg et du bupropion dans une plage posologique de 200 à 450 mg.

11. Composition à utiliser selon l'une quelconque des revendications 1 à 8, comprenant de la trazodone dans une plage posologique de 75 à 150 mg par jour et du bupropion dans une plage posologique de 100 à 300 mg par jour.

12. Composition à utiliser selon l'une quelconque des revendications 1 à 8, comprenant de la trazodone dans une plage posologique de 50 à 100 mg par jour, et du bupropion dans une plage posologique de 100 à 275 mg par jour.

13. Composition à utiliser selon l'une quelconque des revendications 1 à 8, comprenant de la trazodone dans une plage posologique de 75 à 150 mg par jour et du bupropion dans une plage posologique de 225 à 300 mg par jour.

14. Composition à utiliser selon l'une quelconque des revendications 1 à 8, comprenant de la trazodone dans une plage posologique de 50 à 100 mg par jour et du bupropion dans une plage posologique de 200 à 275 mg par jour.

15. Composition à utiliser selon l'une quelconque des revendications 1 à 8, comprenant de la trazodone dans une plage posologique de 25 à 450 mg.

16. Composition à utiliser selon l'une quelconque des revendications 1 à 15, dans laquelle la composition convient pour une administration orale.

17. Composition à utiliser selon l'une quelconque des revendications 1 à 15, dans laquelle la composition convient pour une administration topique.

18. Composition à utiliser selon l'une quelconque des revendications 1 à 15, dans laquelle la composition est administrée par voie orale.

19. Composition à utiliser selon l'une quelconque des revendications 1 à 15, dans laquelle la composition est administrée par voie topique.

20. Composition à utiliser selon l'une quelconque des revendications 1 à 15, dans laquelle le sujet est diagnostiqué et traité pour la dépression.

21. Composition à utiliser selon l'une quelconque des revendications 1 à 15, dans laquelle le sujet ne suit pas de traitement pour la dépression.

22. Composition à utiliser selon l'une quelconque des revendications 1 à 15, dans laquelle le sujet se voit prescrire simultanément un agent thérapeutique additionnel.

23. Composition à utiliser selon l'une quelconque des revendications 1 à 15, dans laquelle le sujet ne se voit pas prescrire simultanément un agent thérapeutique additionnel.

24. Composition à libération prolongée comprenant du bupropion, de la trazodone, et un support pharmaceutiquement acceptable, à utiliser dans le traitement d'un dysfonctionnement sexuel féminin (FSD), d'une baisse du désir sexuel (HSDD), d'un trouble de l'orgasme féminin (FOD), d'un trouble de l'excitation d'intérêt sexuel (FSAD), d'une dyspareunie ou d'un ED chez un sujet.

25. Trousse comprenant une composition comprenant
du bupropion, de la trazodone, et un support pharmaceutiquement acceptable, et
une étiquette fournissant des instructions pour l'administration de la composition à un sujet, à utiliser dans le traitement ou l'amélioration d'une baisse du désir sexuel (HSDD) ou des symptômes de celle-ci chez le sujet.

26. Composition à posologie unique comprenant du bupropion, de la trazodone, et un support pharmaceutiquement acceptable, à utiliser dans le traitement d'un dysfonctionnement sexuel féminin (FSD), d'une baisse du désir sexuel (HSDD), d'un trouble de l'orgasme féminin (FOD), d'un trouble de l'excitation d'intérêt sexuel (FSAD), d'une dyspareunie ou d'un ED chez un sujet.

27. Bupropion et trazodone à utiliser dans le traitement d'un dysfonctionnement sexuel féminin (FSD) chez un sujet.

28. Bupropion et trazodone à utiliser dans le traitement d'une baisse du désir sexuel (HSDD) chez un sujet.

29. Bupropion et trazodone à utiliser dans le traitement d'un trouble de l'orgasme féminin (FOD) chez un sujet.

30. Bupropion et trazodone à utiliser dans le traitement d'un trouble de l'excitation d'intérêt sexuel (FSAD) chez un sujet.

31. Bupropion et trazodone à utiliser dans le traitement d'une dyspareunie chez un sujet.

32. Bupropion et trazodone à utiliser dans le traitement d'un dysfonctionnement érectile (ED) chez un sujet.

33. Bupropion et trazodone à utiliser dans le traitement d'un dysfonctionnement sexuel masculin (MSD) chez un sujet.
